# EUROPEAN PATENT SPECIFICATION

(11) **EP 3 435 942 B1**
(45) Date of publication and mention of the grant of the patent: **20.09.2023**
(21) Application number: 17720873.3
(22) Date of filing: 29.03.2017
(51) Int. Cl.: A61F 13/08, D04B 21/18

(54) **A LIMB COMPRESSION GARMENT FOR APPLYING A COMPRESSIVE FORCE TO A LIMB**
GLIEDMASSENKOMPRESSIONSKLEIDUNGSSTÜCK ZUR APPLIKATION EINER DRUCKKRAFT AUF EINE GLIEDMASSE
VÊTEMENT DE COMPRESSION DE MEMBRE POUR APPLIQUER UNE FORCE DE COMPRESSION SUR UN MEMBRE

(30) Priority: 30.03.2016 GB 201605333
(43) Date of publication of application: 06.02.2019
(73) Proprietor: Haddenham Healthcare Limited, Buckinghamshire HP18 9BB (GB)
(72) Inventor: WRIGHT, Tom, Aylesbury Buckinghamshire HP18 9BB (GB); PÜSCHEL, Frank, 24937 Flensburg (DE)
(74) Representative: Butler, Kathryn Louise
(86) International application number: PCT/IB2017/051785
(87) International publication number: WO 2017/168336

(56) References cited:
- WO-A2-00/06068
- US-A1- 2007 029 308
- US-A1- 2007 179 421
- Nkosilathi Z Nkomo ET AL: "DEVELOPMENT OF A COMPRESSIONAL BANDAGE FOR PROSPECTIVE USE BY PATIENTS", Zimbabwe Journal of Science & Technology, 1 January 2015 (2015-01-01), pages 128-137, XP055376250, Retrieved from the Internet: URL:http://www.nust.ac.zw/zjst/index.php/v olume-10-2015?download=67:development-of-a -compressional-bandage-for-prospective-use -by-patients [retrieved on 2017-05-26]
- Late A. Ruiz-Razura ET AL: "Post Liposuction Compression Garment Comparison: Is There a Difference?", , 23 July 2014 (2014-07-23), pages 1-6, XP055375890, Retrieved from the Internet: URL:https://web.archive.org/web/2014121804 1736/http://www.patientpreferred.com/Compr essionGarmentStudy.pdf [retrieved on 2017-05-24]

## Description

The present invention relates generally to a limb compression garments for applying a compressive force to a limb of a person.

Limb compression garments are used to treat a variety of conditions, most notably swelling and oedema, in which a higher than normal amount of fluid accumulates in the interstitium beneath the skin of a patient.

According to the present invention, there is provided a limb compression garment for applying a compressive force to a limb of a person when wrapped around that limb, the limb compression garment comprising a planar textile short-stretch material comprising: a first plurality of substantially elastic fibres arranged in the plane of the material in a first direction, substantially parallel to each other, the first plurality of substantially elastic fibres being stretchable up to an elastic limit of at least 15% of their unstretched length; and a first plurality of substantially inelastic fibres arranged in the plane of the material, the first plurality of substantially inelastic fibres interlaced with one another to form a textile component, the textile component configured to be extensible in the first direction up to a maximum extension less than the elastic limit; wherein the first plurality of substantially elastic fibres and first plurality of substantially inelastic fibres are interlaced with one another, and wherein the planar short-stretch textile material further comprises a second plurality of substantially inelastic fibres arranged in the plane of the short-stretch material in a second direction, substantially at right angles to the first direction, and substantially parallel to one another, wherein the first plurality of substantially elastic fibres, the first plurality of substantially inelastic fibres and the second plurality of substantially inelastic fibres are interlaced with one another to form, a single-layer short-stretch textile material.

In this way, the textile material is configured to form a "short-stretch" material. The term "short-stretch" is well-understood in the field to mean a material in which an end-of-stretch is reached abruptly at less than approximately 100% of the material's unstretched length.

If the compression garment is worn about a limb at an extension close to the end-of-stretch, then any further swelling that may occur does not result in significant stretching of the garment. That is, at swellings equal to or above the initial swelling present when the compression garment is applied, the garment acts in a similar manner to an entirely inelastic compression garment; however, at swellings below the initial swelling, the material in the garment is free to contact, thereby providing a substantially constant therapeutic compression to the limb.

Compression garments comprising a textile short-stretch material are disclosed in US 2007/0179421 A1; Nkomo et al., "Development of compressional bandage for prospective use by patients", Zimbabwe Journal of Science & Technology, 2015, Vol. 10, 128-137; and Ruiz-Razura et al., "Post liposuction compression garment comparison: is there a difference ?", The Aesthetic Center for Plastic Surgery, 2014.

In the present invention, the first plurality of substantially elastic fibres allows the garment to stretch in the first direction in a substantially elastic manner; however, the first plurality of substantially inelastic fibres abruptly prevents elastic stretch beyond the maximum extension. Textile material may mean any material made of interlacing fibres. The planar textile material may be cloth or fabric, and may comprise natural or artificial/synthetic yarn, thread and/or other fibres. The material may be formed by weaving, knitting, crocheting, knotting, felting, or any other suitable method.

The substantially elastic fibres may comprise rubber, synthetic rubber and/or any other elastic material. Each substantially elastic fibre may obey Hooke's law over a range of extensions up to at least 50% of the length of the substantially elastic fibre, up to at least 100% of the length of the substantially elastic fibre or up to at least 200% of the length of the substantially elastic fibre. Each substantially elastic fibre may resist a distorting stress and return to its original size when the stress is removed, up to its elastic limit. The elastic limit may be at least 20%, 50% or 70% of the unstretched length, in particular at least 100% of the unstretched length, more particularly at least 150% of the unstretched length.

Each substantially inelastic fibre may only obey Hooke's law over a range of extensions up to at most 15% of the length of the substantially elastic fibre, up to at most 10% of the length of the substantially elastic fibre or up to at most 5% of the length of the substantially elastic fibre.

The textile component may be extensible in that each fibre may uncoil, unwind and/or straighten as it is pulled such that the uncoiled, unwound and/or straightened length is longer than the coiled, wound and/or original length before pulling. The maximum extension of each fibre may be reached when it is substantially entirely uncoiled, unwound or straightened. The maximum extension may be between 15% and 100% extension, in particular between 20% and 90 % extension, more particularly between 30% and 70% extension, compared to the unextended length.

The first plurality of substantially inelastic fibres may be interlaced with one another in a warp knit pattern, in which each one of the first plurality of substantially inelastic fibres zigzags along the length of the fabric; that is, following adjacent columns/wales. In particular, the first plurality of substantially inelastic fibres may be interlaced with one another in a tricot pattern; however, other patterns are also envisaged.

The planar textile material may be configured to apply a compression of at least 1kPa, 1.3kPa, 2kPa, 2.7kPa, 3.3kPa, 4kPa, 5.3kPa, 6.7kPa, 8kPa, or 11.4kPa when wrapped around a limb at maximum extension.

In this way any cross-stretch may be limited, thus providing more support to the limb. The first direction may be termed the length of the textile material, and/or the machine direction. The first plurality of substantially elastic fibres may be the warp. The second direction may be termed the width of the material. The second plurality of substantially inelastic fibres may be the weft. Alternatively, the length and width and/or the warp and weft may be swapped, and/or the textile may have no warp and/or weft.

In the context of this patent specification, at right angles may mean between 75 and 105 degrees, in particular between 80 and 100 degrees, more particularly between 85 and 95 degrees.

The first plurality of substantially elastic fibres and/or the second plurality of substantially inelastic fibres may be laid one atop the other, and may not be woven together.

The textile component may merely be a subset of the fibres used to form the textile material, and may not exist separately or independently of the first plurality of substantially elastic fibres and/or the second plurality of substantially inelastic fibres.

The planar textile material may further comprise a third plurality of substantially inelastic fibres loosely interlaced with the first plurality of substantially elastic fibres and/or the first and/or second pluralities of substantially inelastic fibres to form a single-layer textile material. The third plurality of substantially inelastic fibres may substantially cover a first side of the planar textile material.

In this way, a relatively soft texture (compared to the first plurality of substantially elastic fibres and the first and second pluralities of substantially inelastic fibres) may be presented to a user to increase comfort.

The term loosely may refer to the interlacing of the fibres not under tension either in the material's unextended state, and/or in the material's extended state.

The third plurality of substantially inelastic fibres may be oriented in the first direction, or any other desired direction, and may be substantially parallel to one another (although other arrangements are also envisaged).

The planar textile material may further comprise a fourth plurality of substantially inelastic fibres loosely interlaced in the single-layer textile material. The fourth plurality of substantially inelastic fibres may substantially cover a second side of the planar textile material opposite the first side.

In this way, loops may be formed configured to receive corresponding hooks, thereby forming a hook and loop fastening arrangement. Corresponding hooks may be affixed to a portion of the material for self-fastening thereto.

The fourth plurality of substantially inelastic fibres may be oriented in the first direction, or any other desired direction, and may be substantially parallel to one another (although other arrangements are also envisaged).

The fourth plurality of substantially inelastic fibres may be substantially stronger than the third plurality of substantially inelastic fibres.

The planar textile material may be provided in the form of a band for wrapping around a limb of a patient. For instance, the garment may be a bandage.

The band may comprise a fastener at one end thereof for coupling to an outer surface of the band to secure the band in place. The fastener may be a hook, pin or clip, and preferably is a conventional hook part of a hook-and-loop fastening.

For instance, they may be coupled together substantially parallel to one another. The limb compression garment may comprise a plurality of said bands coupled together. In particular, each of said plurality of said bands may be arranged from wrapping around a different portion of a limb of a user. For instance, one band may wrap around a portion of a leg above the knee of a user and another band may wrap around a portion of a leg below the knee of a user. Alternatively, one band may wrap around a portion of an arm immediately adjacent to a portion of the arm about which an adjacent band may wrap. As a further alternative, one band may at least partially overlap an adjacent band.

The limb compression garment may further comprise a stabilisation band, of a different construction to the band discussed above, for wrapping around a limb of a patient. Such stabilisation bands may be conventional bands, such as non-woven, laminated, neoprene, or similar, materials.

The stabilisation band may be shaped to conform to a body part such as a foot, knee or elbow joint. The stabilisation band may have a shape similar to those in known joint supports. The stabilisation band may have a hole therethrough for location over the joint of a user. The stabilisation band may include foam, for instance sewn into the band, for instance around a periphery of the hole.

The garment may comprise at least one band adjacent to the stabilisation band. The garment may comprise at least two bands, one located on either side of the stabilisation band, to wrap around opposing sides of a joint of the user. Further bands may be provided adjacent to one of the bands, distal from the stabilisation band.

The bands may be substantially rectangular in shape, or may have other shapes, including having notches removed therefrom to assist in conforming to a limb.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.
Figure 1 is a plan view of a first subset of fibres in a textile material.
Figure 2 is a plan view of a second subset of fibres in the textile material.
Figure 3 is a plan view of a third subset of fibres in the textile material.
Figure 4 is a plan view of the third subset of fibres in the textile material as the textile material is being stretched.
Figure 5 is a plan view of a fourth subset of fibres in the textile material.
Figure 6 is a plan view of a fifth subset of fibres in the textile material.
Figure 7 is a plan view of a first compression garment.
Figure 8 is a plan view of a second compression garment.

The present invention will be described with respect to certain drawings but the invention is not limited thereto but only by the claims. The drawings described are only schematic and are non-limiting. Each drawing may not include all of the features of the invention and therefore should not necessarily be considered to be an embodiment of the invention. In the drawings, the size of some of the elements may be exaggerated and not drawn to scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that operation is capable in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that operation is capable in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "an embodiment" or "an aspect" means that a particular feature, structure or characteristic described in connection with the embodiment or aspect is included in at least one embodiment or aspect of the present invention. Thus, appearances of the phrases "in one embodiment", "in an embodiment", or "in an aspect" in various places throughout this specification are not necessarily all referring to the same embodiment or aspect, but may refer to different embodiments or aspects. Furthermore, the particular features, structures or characteristics of any embodiment or aspect of the invention may be combined in any suitable manner, as would be apparent to one of ordinary skill in the art from this disclosure, in one or more embodiments or aspects.

Similarly, it should be appreciated that in the description various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Moreover, the description of any individual drawing or aspect should not necessarily be considered to be an embodiment of the invention. Rather, as the following claims reflect, inventive aspects lie in fewer than all features of a single foregoing disclosed embodiment. Thus, the claims following the detailed description are hereby expressly incorporated into this detailed description, with each claim standing on its own as a separate embodiment of this invention.

In the description provided herein, numerous specific details are set forth. However, it is understood that embodiments of the invention may be practised without these specific details. In other instances, well-known methods, structures and techniques have not been shown in detail in order not to obscure an understanding of this description.

In the discussion of the invention, unless stated to the contrary, the disclosure of alternative values for the upper or lower limit of the permitted range of a parameter, coupled with an indication that one of said values is more highly preferred than the other, is to be construed as an implied statement that each intermediate value of said parameter, lying between the more preferred and the less preferred of said alternatives, is itself preferred to said less preferred value and also to each value lying between said less preferred value and said intermediate value.

The use of the term "at least one" may mean only one in certain circumstances.

The principles of the invention will now be described by a detailed description of at least one drawing relating to exemplary features of the invention. It is clear that other arrangements can be configured according to the knowledge of persons skilled in the art without departing from the underlying concept or technical teaching of the invention, the invention being limited only by the terms of the appended claims.

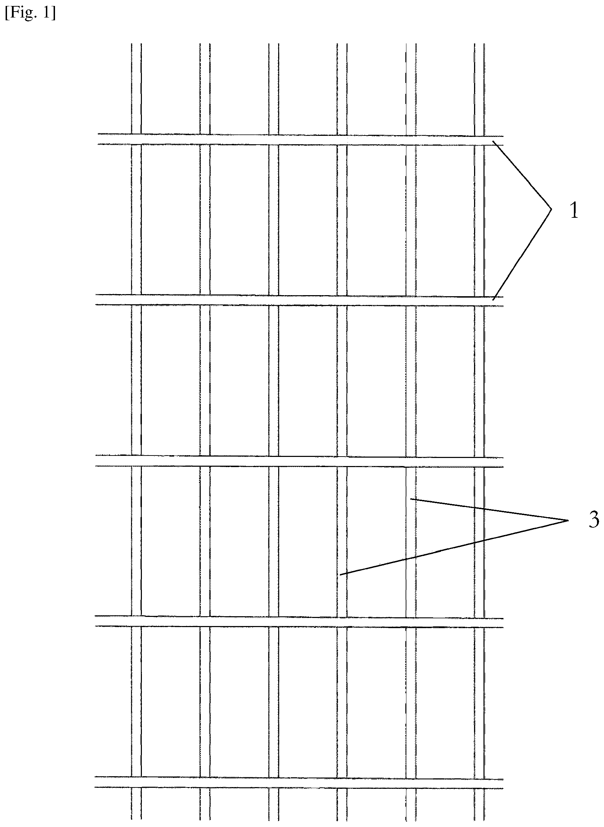

Figure 1 is a plan view of a first subset of fibres in a textile material. A first plurality of substantially elastic fibres 1 are arranged substantially parallel to each other in a first direction. A second plurality of substantially inelastic fibres 3 are arranged beneath the first plurality of substantially elastic fibres 1, substantially at right angles thereto. In this arrangement, the first plurality of substantially elastic fibres 1 and the second plurality of substantially inelastic fibres 3 do not interlace, but are rather merely laid one atop the other; however, arrangements in which they interlace, for instance being weaved together, are envisaged.

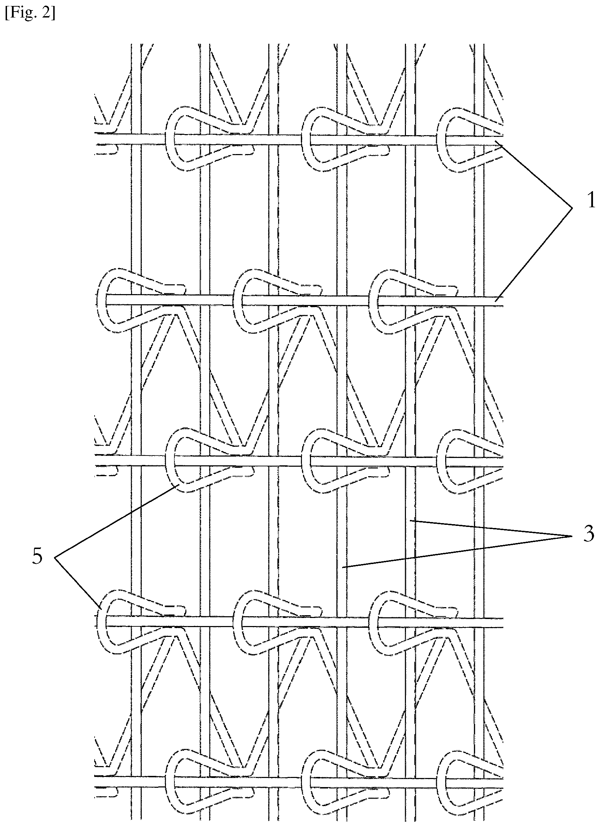

Figure 2 is a plan view, similar to that of figure 1, but also showing additional inelastic fibres 5 zigzagging between adjacent ones of the first plurality of substantially elastic material. It is to be noted that figures 1 to 3 do not show the process by which the textile material is formed, but merely illustrates its construction by identifying additional components in sequential figures,

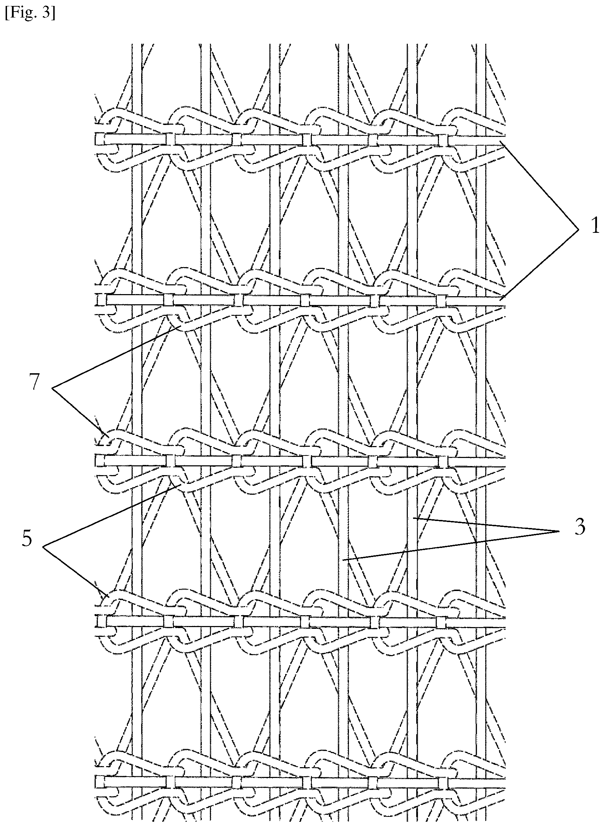

Figure 3 is a plan view similar to that of figures 1 and 2, but also showing further inelastic fibres 7. Together the inelastic fibres 5 and the further inelastic fibres 7 form a first plurality of substantially inelastic fibres that interlace to form a secure textile component into which the plurality of substantially elastic fibres 1 and the second plurality of substantially inelastic fibres 3 are interlaced. Figure 3 shows a tricot pattern, but other patterns are also envisaged.

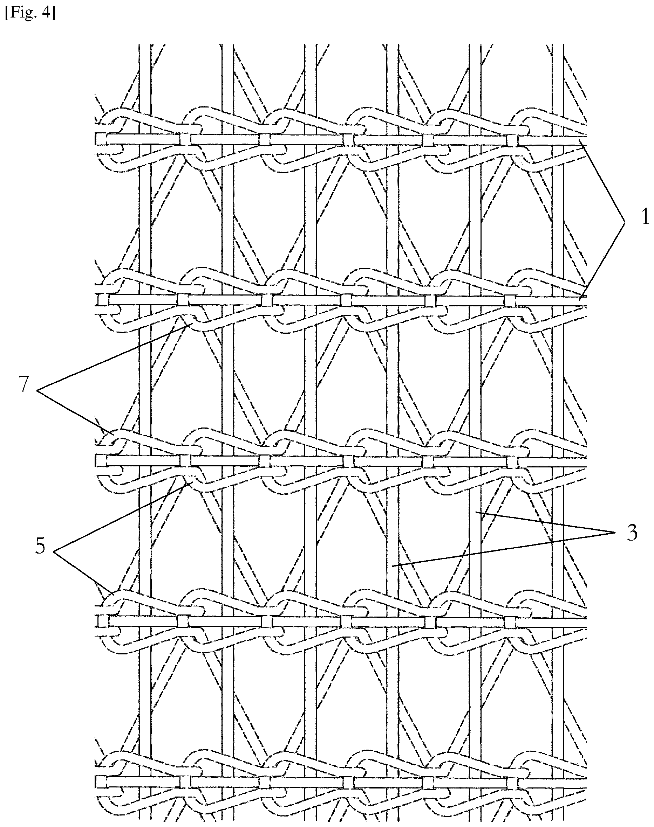

Figure 4 is a plan view showing the configuration of the textile material of figure 3 when being stretched along the axis of the first plurality of substantially elastic fibres 1.

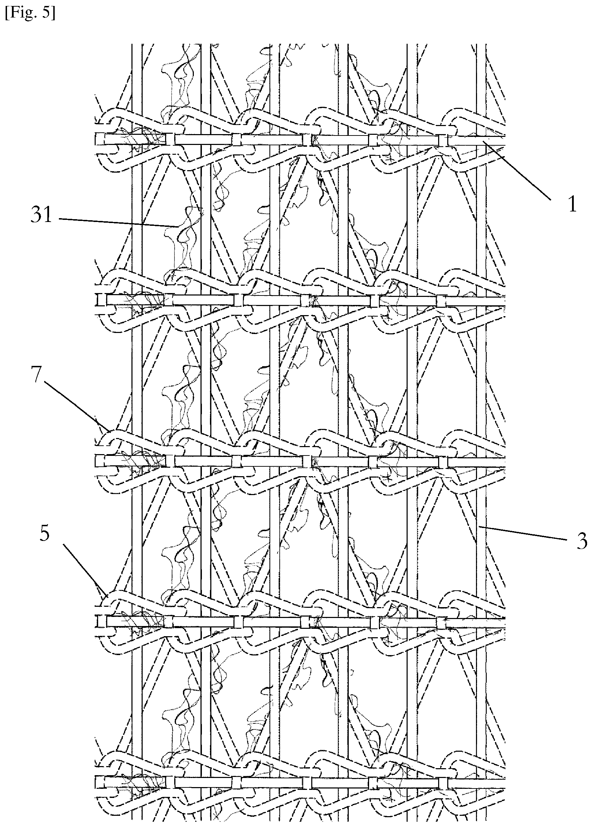

Figure 5 is a plan view similar to that of figures 1, 2 and 3, but also showing further inelastic fibres 31. The fibres 31 may form a strand, but may not be yarn/thread, in that they may not be interlocked by twisting and/or braiding, but rather comprise a plurality of individual fibres loosely arranged parallel to one another, as in the figure. Such loose arrangement may be loose interlocking. In particular, the fibres 31 may be looped, curved and/or otherwise substantially non-straight fibres. The fibres may therefore comprise a series of loops that act as a loop part of a hook-and-loop fastener.

The fibres 31 may be interlocked into the planar textile material substantially on an outside of the material; that is, a greater proportion of the length of the fibres 31 is located on the outside surface of the material, with a smaller proportion of the length of the fibres 31 passing behind one or more other fibres in the material. For instance, in the present example, the fibres 31 are located substantially on the far side of the material except at various locations where the fibres 31 pass in front of the elastic fibres 1; however, other interlocking arrangements are also envisaged.

The fibres 31 shown in the figure are arranged in a zig-zag arrangement, for instance, such that they trace out a path that runs parallel to a first one of the elastic fibres 1 past three loops of the inelastic fibres 5, 7, loops behind the first elastic fibre 1 to interlock therewith, then diagonally across to an adjacent second one of the elastic fibre 1, loops behind the second elastic fibre 1 to interlock therewith, then diagonally back past the first elastic fibre 1 to a further adjacent third one of the elastic fibres 1, and finally loops behind the third elastic fibre 1 to interlock therewith, before repeating the pattern. It is to be understood that there are a large variety of different patterns that may be chosen for the fibres 31.

The fibres 31 may comprise polyamide.

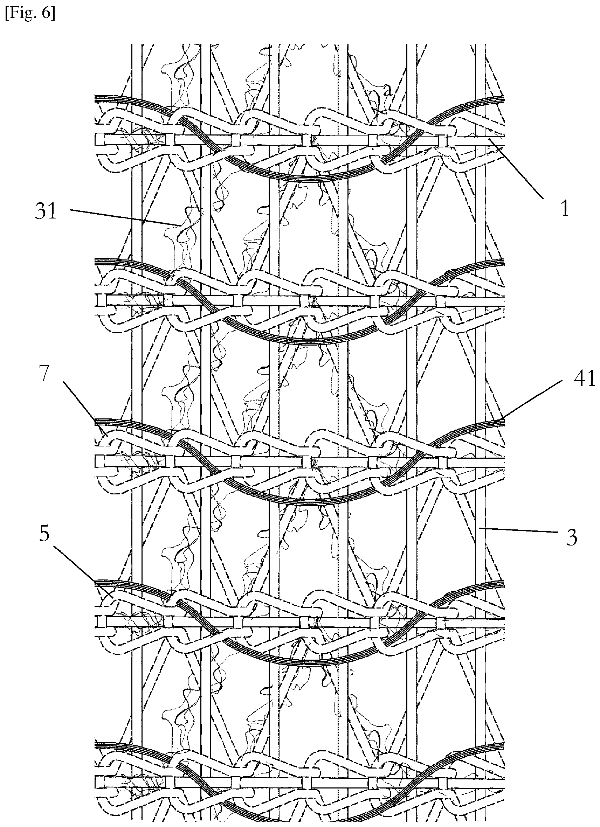

Figure 6 is a plan view similar to that of figures 1, 2, 3 and 5, but also showing still further inelastic fibres 41, loosely interlaced with the first and second inelastic fibres 5, 7; though other interlacing patterns are envisaged. The inelastic fibres 41 are arranged substantially parallel to the elastic fibres 1, but are shown curving for the purpose of clarity. The fibres 41 are located substantially on the inside (i.e. the front face) of the material, merely looping behind the loops in the inelastic fibres 5, 7 to interlace with the material; however, other interlacings are also envisaged, such as behind the inelastic fibres 3.

The fibres 41 are shown looping behind every third loop of the inelastic fibres 5, 7; however, in various arrangements, any frequency of interlocking (such as every, every second, every fourth, every fifth or every sixth, etc. loop) is also envisaged.

Each strand of fibres 41 is shown in the figure as 5 parallel lines to indicate that each strand may not be yarn/thread, as discussed above.

The fibres 41 may be chosen to feel soft against the skin of a user. The density of fibres may be such that they substantially obscure the remainder of the material from casual inspection, when viewed from the inside.

The fibres 41 may be loose, as defined above. The third plurality of substantially inelastic fibres may be substantially weaker than the fourth plurality of substantially inelastic fibres.

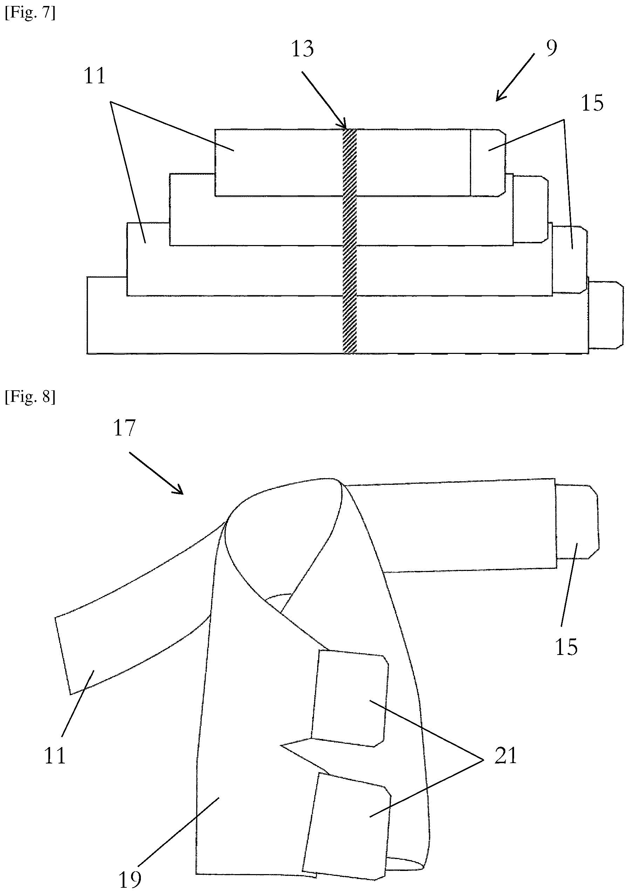

Figure 7 is a plan view of a first compression garment 9 in which a plurality of bands 11 are connected together along a central spine 13. The central spine 13 may simply be stitching connecting adjacent bands 11 to one another. Alternatively, the spine 13 may be an independent substrate to which the bands 11 are affixed. At the end of each band 11 is a hook fastener 15 (of the hook-and-loop type fastener) that is configured to engage with corresponding loop material present in the surface of the bands 11. As can be seen from the figures, the bands 11 are of various different lengths, chosen to fit a limb of a user. For instance, the smallest band 11 may be wrapped around a leg above the malleolus of a user, while the largest band 11 may be wrapped around the user's leg adjacent to the head of the fibula, with the intervening bands 11 wrapped around intermediate portions of the user's forearm. In particular, the size of each band 11 may be chosen to match the circumference of the portion of the limb about which they are to be wrapped.

Figure 8 is a plan view of a second compression garment 17 in which a single band 11 is configured to wrap around a user's ankle, and be secured in place by fastener 15. Coupled to the band 11 is a foot stabilisation part 19 formed of a neoprene material and configured to be wrapped around the foot of a user, leaving the toes to project therefrom. The foot stabilisation part 19 may be tightened and secured in place by further hook fasteners 21 in a conventional manner.

## Claims

1. A limb compression garment for applying a compressive force to a limb of a person when wrapped around that limb, the limb compression garment comprising a planar textile short-stretch material comprising:
a first plurality of substantially elastic fibres (1) arranged in the plane of the short-stretch material in a first direction, substantially parallel to each other, the first plurality of substantially elastic fibres being stretchable up to an elastic limit of at least 15% of their unstretched length; and
a first plurality of substantially inelastic fibres (5, 7) arranged in the plane of the short-stretch material, the first plurality of substantially inelastic fibres interlaced with one another to form a textile component, the textile component configured to be extensible in the first direction up to a maximum extension less than the elastic limit;
wherein the first plurality of substantially elastic fibres and first plurality of substantially inelastic fibres are interlaced with one another to form a single-layer short-stretch textile material, and wherein the planar short-stretch textile material further comprises a second plurality of substantially inelastic fibres (3) arranged in the plane of the short-stretch material in a second direction, substantially at right angles to the first direction, and substantially parallel to one another, wherein the first plurality of substantially elastic fibres, the first plurality of substantially inelastic fibres and the second plurality of substantially inelastic fibres are interlaced with one another to form a single-layer short-stretch textile material..

2. The limb compression garment of claim 1, wherein the planar textile short-stretch material further comprises a plurality of substantially inelastic outer fibres loosely interlaced in the single-layer short-stretch textile material, wherein the plurality of substantially inelastic outer fibres substantially cover an outside of the planar short-stretch textile material.

3. The limb compression garment of claim 1 or claim 2, wherein the planar short-stretch textile material further comprises a plurality of substantially inelastic inner fibres loosely interlaced in the single-layer short-stretch textile material, wherein the third plurality of substantially inelastic fibres substantially cover an inside of the planar short-stretch textile material.

4. The limb compression garment of any preceding claim, wherein the planar short-stretch textile material is configured to apply a compression of at least 1kPa when wrapped around a limb at maximum extension.

5. The limb compression garment of any preceding claim, wherein the planar short-stretch textile material is provided in the form of a band for wrapping around a limb of a patient.

6. The limb compression garment of claim 5, wherein the band comprises a fastener at one end thereof for coupling to an outer surface of the band to secure the band in place.

7. The limb compression garment of claim 5 or claim 6, comprising a plurality of said bands coupled together.

8. The limb compression garment of any one of claims 5 to 7, further comprising a stabilisation band, of a different construction to the band of any one of claims 5 to 7, for wrapping around a limb of a patient.

## Patentansprüche

1. Gliedmaßenkompressionskleidungsstück zur Applikation einer Druckkraft auf eine Gliedmaße einer Person, wenn es um die Gliedmaße gewickelt ist, das Gliedmaßenkompressionskleidungsstück umfassend ein ebenes Textilmaterial mit kurzer Dehnung, umfassend:
eine erste Vielzahl von im Wesentlichen elastischen Fasern (1), die auf der Ebene des Materials mit kurzer Dehnung in einer ersten Richtung im Wesentlichen parallel zueinander angeordnet sind, wobei die erste Vielzahl von im Wesentlichen elastischen Fasern bis zu einer Elastizitätsgrenze von mindestens 15 % ihrer ungedehnten Länge dehnbar ist,
und
eine erste Vielzahl von im Wesentlichen unelastischen Fasern (5, 7), die auf der Ebene des Materials mit kurzer Dehnung angeordnet sind, wobei die erste Vielzahl von im Wesentlichen unelastischen Fasern miteinander verflochten sind, um eine Textilkomponente zu bilden, wobei die Textilkomponente dazu konfiguriert ist, in der ersten Richtung bis zu einer maximalen Dehnung kleiner als die Elastizitätsgrenze ausdehnbar zu sein;
wobei die erste Vielzahl von im Wesentlichen elastischen Fasern und die erste Vielzahl von im Wesentlichen unelastischen Fasern miteinander verflochten sind, um ein einschichtiges Textilmaterial mit kurzer Dehnung zu bilden, und wobei das ebene Textilmaterial mit kurzer Dehnung ferner eine zweite Vielzahl von im Wesentlichen unelastischen Fasern (3) umfasst, die auf der Ebene des Materials mit kurzer Dehnung in einer zweiten Richtung im Wesentlichen im rechten Winkel zu der ersten Richtung und im Wesentlichen parallel zueinander angeordnet sind, wobei die erste Vielzahl von im Wesentlichen elastischen Fasern, die erste Vielzahl von im Wesentlichen unelastischen Fasern und die zweite Vielzahl von im Wesentlichen unelastischen Fasern miteinander verflochten sind, um ein einlagiges Textilmaterial mit kurzer Dehnung zu bilden.

2. Gliedmaßenkompressionskleidungsstück nach Anspruch 1, wobei das ebene Textilmaterial mit kurzer Dehnung ferner eine Vielzahl von im Wesentlichen unelastischen Außenfasern umfasst, die lose in dem einschichtigen Textilmaterial mit kurzer Dehnung verflochten sind, wobei die Vielzahl von im Wesentlichen unelastischen Außenfasern eine Außenseite des ebenen Textilmaterials mit kurzer Dehnung im Wesentlichen bedecken.

3. Gliedmaßenkompressionskleidungsstück nach Anspruch 1 oder Anspruch 2, wobei das ebene Textilmaterial mit kurzer Dehnung ferner eine Vielzahl von im Wesentlichen unelastischen Innenfasern umfasst, die lose in dem einschichtigen Textilmaterial mit kurzer Dehnung verflochten sind, wobei die dritte Vielzahl von im Wesentlichen unelastischen Fasern eine Innenseite des ebenen Textilmaterials mit kurzer Dehnung im Wesentlichen bedecken.

4. Gliedmaßenkompressionskleidungsstück nach einem der vorhergehenden Ansprüche, wobei das ebenes Textilmaterial mit kurzer Dehnung dazu konfiguriert ist, eine Kompression von mindestens 1 kPa zu applizieren, wenn es bei maximaler Ausdehnung um eine Gliedmaße gewickelt ist.

5. Gliedmaßenkompressionskleidungsstück nach einem der vorhergehenden Ansprüche, wobei das ebene Textilmaterial mit kurzer Dehnung in Form eines Bandes zum Umwickeln einer Gliedmaße eines Patienten bereitgestellt ist.

6. Gliedmaßenkompressionskleidungsstück nach Anspruch 5, wobei das Band an einem Ende davon ein Befestigungsmittel zum Koppeln an eine Außenfläche des Bandes umfasst, um das Band an Ort und Stelle zu sichern.

7. Gliedmaßenkompressionskleidungsstück nach Anspruch 5 oder Anspruch 6, umfassend eine Vielzahl der aneinander gekoppelten Bänder.

8. Gliedmaßenkompressionskleidungsstück nach einem der Ansprüche 5 bis 7, ferner umfassend ein Stabilisierungsband mit einer anderen Konstruktion als das Band nach einem der Ansprüche 5 bis 7 zum Umwickeln einer Gliedmaße eines Patienten.

## Revendications

1. Vêtement de compression de membre destiné à appliquer une force de compression sur un membre d'une personne lorsque il est enroulé autour de ce membre, le vêtement de compression de membre comprenant un matériau textile plat à étirement court comprenant :
une première pluralité de fibres sensiblement élastiques (1) agencées dans le plan du matériau plat à étirement court dans une première direction, sensiblement parallèles les unes aux autres, la première pluralité de fibres sensiblement élastiques étant étirables jusqu'à une limite élastique supérieure ou égale à 15 % de leur longueur non étirée ; et
une première pluralité de fibres sensiblement inélastiques (5, 7), agencées dans le plan du matériau à étirement court, la première pluralité de fibres sensiblement inélastiques étant entrelacées les unes avec les autres pour former un composant textile, le composant textile étant conçu pour être extensible dans la première direction jusqu'à une extension maximale inférieure à la limite élastique ;
ladite première pluralité de fibres sensiblement élastiques et ladite première pluralité de fibres sensiblement inélastiques étant entrelacées les unes avec les autres pour former un matériau textile à étirement court monocouche, et ledit matériau textile plat à étirement court comprenant en outre une deuxième pluralité de fibres sensiblement inélastiques (3) agencées dans le plan du matériau à étirement court dans une seconde direction, sensiblement à angle droit par rapport à la première direction, et sensiblement parallèles les unes aux autres, ladite première pluralité de fibres sensiblement élastiques, ladite première pluralité de fibres sensiblement inélastiques et ladite deuxième pluralité de fibres sensiblement inélastiques étant entrelacées les unes avec les autres pour former un matériau textile à étirement court monocouche.

2. Vêtement de compression de membre selon la revendication 1, ledit matériau textile plat à étirement court comprenant en outre une pluralité de fibres externes sensiblement inélastiques entrelacées de manière lâche dans le matériau textile à étirement court monocouche, ladite pluralité de fibres externes sensiblement inélastiques recouvrant sensiblement un extérieur du matériau textile plat à étirement court.

3. Vêtement de compression de membre selon la revendication 1 ou la revendication 2, ledit matériau textile plat à étirement court comprenant en outre une pluralité de fibres internes sensiblement inélastiques entrelacées de manière lâche dans le matériau textile à étirement court monocouche, ladite troisième pluralité de fibres sensiblement inélastiques recouvrant un intérieur du matériau textile plat à étirement court.

4. Vêtement de compression de membre selon une quelconque revendication précédente, ledit matériau textile plat à étirement court étant conçu pour appliquer une compression supérieure ou égale à 1 kPa lorsqu'il est enroulé autour d'un membre à une extension maximale.

5. Vêtement de compression de membre selon une quelconque revendication précédente, ledit matériau textile plat à étirement court étant prévu sous la forme d'une bande destinée à s'enrouler autour d'un membre d'un patient.

6. Vêtement de compression de membre selon la revendication 5, ladite bande comprenant un élément de fixation au niveau d'une extrémité de celle-ci pour se coupler à une surface externe de la bande pour fixer la bande en place.

7. Vêtement de compression de membre selon la revendication 5 ou la revendication 6, comprenant une pluralité desdites bandes couplées ensemble.

8. Vêtement de compression de membre selon l'une quelconque des revendications 5 à 7, comprenant en outre une bande de stabilisation, d'une construction différente de la bande de l'une quelconque des revendications 5 à 7, destinée à s'enrouler autour d'un membre d'un patient.
